# EUROPEAN PATENT SPECIFICATION

(11) **EP 3 648 734 B1**
(45) Date of publication and mention of the grant of the patent: **06.08.2025**
(21) Application number: 18735271.1
(22) Date of filing: 28.06.2018
(51) Int. Cl.: A61K 8/37, A61Q 17/00, A61P 17/10, A61Q 19/00, A61K 8/35, A61K 8/44, A61Q 5/00, A61K 8/60

(54) **TOPICAL COMPOSITIONS**
TOPISCHE ZUSAMMENSETZUNGEN
COMPOSITIONS TOPIQUES

(30) Priority: 06.07.2017 EP 17180058
(43) Date of publication of application: 13.05.2020
(73) Proprietor: DSM IP Assets B.V., 6221 BE Maastricht (NL)
(72) Inventor: MENDROK-EDINGER, Christine, 4303 Kaiseraugst (CH); MONGIAT, Sebastien, 4303 Kaiseraugst (CH); RUDOLPH, Thomas, 4303 Kaiseraugst (CH)
(74) Representative: dsm-firmenich IP
(86) International application number: PCT/EP2018/067369
(87) International publication number: WO 2019/007791

(56) References cited:
- EP-A2- 0 221 728
- US-A1- 2004 062 781
- US-A1- 2006 177 392
- US-B1- 8 747 818

## Description

The present invention relates to topical compositions comprising erythrulose and retinyl acetate,

US2006/177392 discloses an oil-based topical composition for use on the skin containing at least one compound from the class of retinoids such as retinyl esters, which are useful as medicinal agents, in an oleaginous solution composed substantially of non-ionic lipids, which are useful as vehicles for nonpolar compounds, e.g. for the treatment of acne.

US2004062781 relates to a new retinyl retinoate, a process for preparing same, a new intermediate which is useful for preparing same, and a cosmetic composition comprising the retinyl retinoate as the active ingredient for the treatment e.g. of skin ageing and acne.

EP0221728 discloses an antimicrobial activity of erythrulose.

Surprisingly it has now been found that erythrulose, a well-accepted cosmetic ingredient synergistically enhances the antimicrobial activity of the retinylester retinyl acetate against fungi and/ or bacteria. Thus, the combination can effectively be used to balance the skin microbiome and overcome adverse effects resulting from microbial overpopulation on the skin.

Thus, in a first embodiment the present invention relates to topical compositions comprising erythrulose and retinyl

In another embodiment, the invention relates to the use of a mixture of erythrulose and retinyl acetate, as antimicrobial agent such as preferably for the inhibition of the growth of E. coli and/ or P. acnes.

In a further embodiment, the invention relates to a method of killing and/ or inhibiting growth of microbial cells, in particular fungal and/ or bacterial cells, said method comprising contacting said cells with erythrulose, with a mixture of erythrulose and retinyl acetate,

Due to the antimicrobial activity against fungal and/ or bacterial cells the mixture of erythrulose and retinyl acetate, is further suitable for the treatment of adverse skin conditions associated with an overpopulation of such fungal and/ or bacterial cells by maintaining skin homeostasis and/ or improving the health of the skin microbiome.

Further suitable uses of the topical compositions according to the present invention encompass pharmaceutical applications. Thus, the topical compositions according to the present invention may be used for the treatment, prevention and/or prophylaxis of any disorder and disease where it is desirable to kill and/ or inhibit the growth of fungal and/ or bacterial cells.

The term "erythrulose" refers to erythrulose in D- or L-form or as the racemate. Preferably L-(+)-Erythrulose [533-50-6] is used. Erythrulose is e.g. commercially available at DSM Nutritional Products Ltd, Kaiseraugst.

The retinylesters according to the present invention are known compounds and commercially available. Retinyl acetate is e.g. commercially available at DSM Nutritional Products as Vitamin A Acetate 1.5 MIU/g. It contains 1 500 000 IU pure vitamin A in the form of acetate (450 000 µg retinol) per gram in peanut oil

The term "antimicrobial activity" (or "antimicrobial effect") as used herein means a capability of killing and/or inhibiting the growth of microbial cells such as fungal or bacterial cells, such as in particular P. acnes, S. epidermis, C. xerosis, A. brasiliensis, C. albicans, P. aeruginosa, E. coli, M. furfur and/ or S. aureus such as in particular P. acnes and E. coli.

In all embodiments of the present invention the topical compositions preferably comprise erythrulose in an amount selected in the range of 0.005 to 5 wt.-%, more preferably in the range of 0.01 to 3 wt.-% and most preferably in the range of 0.025 to 2 wt.-%, such as in an amount of 0.04 to 1 wt.-% and particularly advantageous in an amount of 0.04 to 0.75 wt.-%, based on the total weight of the composition. Further suitable ranges of erythrulose encompass 0.04 to 0.2 wt.-% and 0.05 to 0.1 wt.-%.

In all embodiments of the present invention the topical compositions preferably comprise the retinylester in an amount selected in the range of 0.00001 to 1 wt.-%, preferably in the range of 0.0001 to 0.75 wt.-%, most preferably in the range of 0.001 to 0.5 wt.-%, based on the total weight of the composition. Further suitable ranges encompass 0.001 to 0.2 wt.-% and 0.001 to 0.1 wt.-%.

The topical composition according to the present invention are particularly used in the treatment, prevention and/or prophylaxis of acne as the combination of combination of erythrulose and retinyl acetate acts synergistically on the microbial cells responsible thereof, i.e. P. acnes.

As the combination of erythrulose and retinyl acetate also acts synergistically against E. coli, which knowingly causes the degradation (microbial breakdown) of products such as e.g. cosmetic products, the present invention is also concerned with the use of a combination of erythrulose and retinyl acetate and with all the definitions and preferences as given herein for improving preservation of a product selected from the group of cosmetic compositions, household products, plastics, paper and/ or paints.

In a further embodiment, the present invention is directed to a method for improving the preservation of a product selected from the group of cosmetic compositions, household products, plastics, paper and/ or paints, said method encompassing the step of adding erythulose in combination with retinyl acetate and with all the definitions and preferences as given herein to said product.

It is well understood that the improved preservation is compared to a product not containing erythrulose and a retinylester, respectively only one of them.

In a further embodiment the present invention is related with a method of preventing microbial decay and breakdown caused by E. coli of cosmetic compositions, household products, plastics, paper and/ or paints, wherein said method comprises adding to the cosmetic compositions, household products, plastics, paper and/ or paints erythrulose and retinyl acetate with all the definitions and preferences as given herein.

To make use of the anti-microbial activity of the combination of erythrulose and retinyl acetate, it can be used in a multiplicity of formulations or applications, such as, for example, cosmetic or pharmaceutical compositions, medicinal products or household products.

The use according to the invention of the combination of erythrulose and retinyl acetate, can take place both in the cosmetic sense as well as in the pharmaceutical sense or even in a technical sense e.g. for the preservation of paints. A pharmaceutical application is conceivable, for example, in the case of anti-dandruff or anti-acne compositions. In all embodiments of the present invention, the use is however preferably cosmetic (non-therapeutic) such as for maintenance of skin homeostasis and/ or balancing the skin microbiome, i.e. maintaining a healthy skin microbiome.

The topical compositions according to the present invention are preferably cosmetic or pharmaceutical compositions which are topically applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The cosmetic or pharmaceutical compositions according to the present invention preferably further comprise a physiologically acceptable medium, that is to say a medium compatible with keratinous substances, such as the skin, mucosa, and keratinous fibers. Preferably, the physiologically acceptable medium is a cosmetically or pharmaceutically acceptable carrier.

The term cosmetically or pharmaceutically acceptable carrier refers to all carriers and/or excipients and/ or diluents conventionally used in cosmetic compositions.

The topical compositions according to the present invention are generally prepared by admixing erythrulose and retinyl acetate, in the amounts indicated herein with a suitable carrier.

The exact amount of carrier will depend upon the actual level of erythrulose and the retinylester and any other optional ingredients that one of ordinary skill in the art would classify as distinct from the carrier (e.g., other active ingredients).

In an advantageous embodiment, the cosmetic or pharmaceutical compositions according to the present invention comprise from 50% to 99%, preferably from 60% to 98%, more preferably from 70% to 98%, such as in particular from 80% to 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic or pharmaceutical compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Preferably, the cosmetic or pharmaceutical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

The cosmetic or pharmaceutical compositions in accordance with the invention can be in the form of a liquid, lotion, a thickened lotion, a gel, a cream, a milk, an ointment or a paste.

The cosmetic or pharmaceutical compositions according to the invention have a pH in the range of 3-10, preferably in the range of pH of 3-8, most preferred in the range of pH 3-6.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention citric acid in an amount of at least 0.0001 wt.-%, such as e.g. in an amount of 0.01-1 wt.-%, in particular in an amount of 0.01 to 0.5 wt.-% is used for pH adjustment.

The cosmetic compositions according to the present invention advantageously comprise a preservative. Particular suitable preservatives in all embodiments of the present invention are phenoxyethanol and ethylhexylglycerin as well as mixtures thereof. When present, the preservative is preferably used in an amount of 0.01 to 2 wt.-%, more preferably in an amount of 0.05 to 1.5 wt.-%, most preferably in an amount of 0.1 to 1.0 wt.-%, based on the total weight of the composition.

The cosmetic compositions according to the present invention are in particular skin care preparations, functional preparations and/ or hair care preparations such as most in particularly skin or hair care preparations.

Examples of skin care preparations are, in particular, light protective preparations, anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples hair care preparations which are suitable according to the invention and which may be mentioned are shampoos, hair conditioners (also referred to as hair rinses), hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

If the hair care preparations according to the invention are supplied as shampoos, these can be clear liquids, opaque liquids (with pearly luster effect), in cream form, gel-like or else in powder form or in tablet form, and as aerosols. The surfactant raw materials on which these shampoos are based can be anionic, cationic, nonionic and amphoteric in nature and also be present in combinations of these substances.

Examples of anionic surfactants suitable for the incorporation into the shampoo preparations according to the present invention are C₁₀₋₂₀ alkyl- and alkylenecarboxylates, alkyl ether carboxylates, fatty alcohol sulfates, fatty alcohol ether sulfates, alkylolamide sulfates and sulfonates, fatty acid alkylolamide polyglycol ether sulfates, alkanesulfonates and hydroxyalkanesulfonates, olefinsulfonates, acyl esters of isothionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyltaurides, fatty acid sarcosinates, and sulforicinoleates. These compounds and their mixtures are used in the form of their salts which are soluble in water or dispersible in water, for example the sodium, potassium, magnesium, ammonium, mono-, di- and triethanolammonium and analogous alkylanunonium salts.

Examples of suitable cationic surfactants are quaternary ammonium salts such as di(C₁₀₋-C₂₄alkyl)dimethylammonium chloride or bromide, preferably di (C₁₂-C₁₈alkyl)-dimethylammonium chloride or bromide; C₁₀-C₂₄-alkyldimethylethylammonium chloride or bromide; C₁₀-C₂₄-alkyltrimethylammonium chloride or bromide, preferably cetyltrimethylammonium chloride or bromide and C₂₀-C₂₄-alkyltrimethylammonium chloride or bromide; C₁₀-C₂₄4 -alkyldimethylbenzylammonium chloride or bromide, preferably C₁₂-C₁₈-alkyldime methylbenzylammoniumchloride; N-(C₁₂-C₁₈-alkyl)pyridinium chloride or bromide, preferably N- (C₁₂-C₁₆-alkyl)pyridinium chloride or bromide; N-(C₁₂-C₁₈-alkyl)isoquinolinium chloride, bromide or monoalkyl sulfate; N-(C₁₂-C₁₈-alkyloylcolaminoformylmethyl)pyridinium chloride; N-(C₁₂-C₁₈-alkyl)-N-methylmorpholinium chloride, bromide or monoalkyl sulfate; N-(C₁₂-C₁₈-alkyl)-N-ethylmorpholinium chloride, bromide or monoalkyl sulfate; C₁₆-C₁₈-alkylpentaoxethylammonium chloride; isobutylphenoxyethoxyethyldimethyl-benzylammonium chloride; salts of N,N-diethylaminoethylstearylamide and oleylamide with hydrochloric acid, acetic acid, lactic acid, citric acid, phosphoric acid; N-acylamidoethyl-N,N-diethyl-N-methylammonium chloride, bromide or monoalkylsulfate and N-acylaminoethyl-N,N-diethyl-N-benzylammonium chloride, bromide or monoalkyl sulfate, where acyl is preferably stearyl or oleyl.

Examples of suitable nonionic surfactants which can be used as detergent substances are fatty alcohol ethoxylates (alkylpolyethylene glycols); alkylphenol polyethylene glycols; alkyl mercaptan polyethylene glycols; fattyamine ethoxylates (alkylaminopolyethylene glycols); fatty acid ethoxylates (acyl polyethylene glycols); polypropylene glycol ethoxylates (Pluronic); fatty acid alkylolamides (fatty acid amide polyethylene glycols); sucrose esters; sorbitol esters and polyglycol ether.

Examples of amphoteric surfactants which can be added to the shampoos are N-(C₁₂-C₁₈-alkyl)-.beta.-aminopropionates and N-(C₁₂-C₁₈-alkyl)-.beta.-iminodipropionates as alkali metal and mono-, di- and trialkylammonium salts; N-acylamidoalkyl-N,N-dimethylacetobetaine, preferably N-(C₈-C₁₈-acyl)amidopropyl-N, N-dimethylacetobetaine; C₁₂-C₁₈-alkyldimethylsulfopropylbetaine; amphoteric surfactants based on imidazoline (commercial name: Miranol^{®}, Steinapon^{®}), preferably the sodium salt of 1-(β-carboxymethyloxyethyl)-1-(carboxymethyl)-2-laurylimidazolinium; amine oxide, for example C₁₂-C₁₈-alkyldimethylamine oxide, fatty acid amidoalkyldimethylamine oxide.

The hair care preparations according to the invention can additionally contain further additives customary in hair care such as for example perfumes, colorants, also those which simultaneously dye or tint the hair, solvents, opacifying agents and pearly luster agents, for example esters of fatty acids with polyols, magnesium and zinc salts of fatty acids, dispersions based on copolymers, thickening agents such as sodium, potassium and ammonium chloride, sodium sulfate, fatty acid alkylolamides, cellulose derivatives, natural rubbers, also plant extracts, protein derivatives such as gelatin, collagen hydrolysates, polypeptides with a natural or synthetic basis, egg yolk, lecithin, lanolin and lanolin derivatives, fats, oils, fatty alcohols, silicones, deodorizing agents, substances with antimicrobial activity, substances with antiseborrhoeic activity, substances with keratolytic and keratoplastic effect, such as, for example, sulfur, salicylic acid and enzymes as well as further anti-dandruff agents such as olamine, climbazol, zink pyrithion, ketoconazole, salicylic acid, sulfur, tar preparations, derivatives of undecenic acid, extracts of nettel, rosmary, cottonwood, birch, walnut, willow bark and/ or arnica.

As the compositions according to the present invention are particularly suitable to treat dandruff, the present invention also relates to a method of treating the scalp, said method comprising the steps of contacting the scalp with a hair care preparation comprising erythrulose and a retinylester selected from the group consisting of retinyl acetate, retinyl propionate, retinyl linoleate, retinyl retinoate and/ or retinyl formyl aspartamate, preferably retinyl acetate. In a preferred embodiment the method is directed to the treatment of dandruff. In another preferred embodiment, the hair care preparation is a rinse off composition in the form of a shampoo or a conditioner. In a further preferred embodiment, the method furthermore comprises the step of rinsing the hair with water.

The shampoos are produced in a manner known per se by mixing the individual components and where necessary further processing appropriate for the particular type of preparation.

Examples of hair care preparations in which the combination of erythrulose and hydroxacetophenone can be used according to the invention and which may be mentioned are hair conditioners, hair tonics and hair regenerating compositions, which are rinsed off from the hair after a certain time or, depending on the formulation, can also remain on the hair.

All these preparations are also produced as already mentioned for the shampoo in a manner known per se with the addition of the combination of erythrulose and hydroxacetophenone.

The following examples are provided to further illustrate the compositions and effects of the present invention. These examples are illustrative only and are not intended to limit the scope of the invention in any way.

### Example 1: Synergistic affect against Escherichia coli and Propionibacterium acnes

The antimicrobial efficacy is assessed in analogy to the regulatory challenge test method (NF EN ISO11930). Thus, solutions of the respective active(s) in ethanol are prepared and further dissolved in physiological serum with 0.85 wt.-% NaCl in the concentrations as outlined in table 1 under sterile conditions. The samples were solubilized in physiological serum supplemented with 1 wt.-% ethanol. A control was prepared under sterile condition based on a serum with 0.85 wt.-% NaCl and 1 wt.-% ethanol **(C1).**

The control as well as the solutions of the active(s) were deposed in 96-deep well plates (1.6 ml/well). The wells are contaminated with either Propionibacterium acnes or Escherichia coli at 2.5*10⁵ to 5.6*10⁵ cfu/ml to obtain the initial contamination as outlined in table 1. After the contamination, each well was thoroughly mixed to ensure a homogeneous distribution of bacteria. Then each plate was incubated at 22°C for 24h. The counting of the (remaining) population is carried out 24h after contamination.

**Table 1: Results on P. acnes**

| Test solution | Time [h] | Propionibacterium acnes colony count [cfu/ml] | Log reduction |
|---|---|---|---|
| 0.1 wt.-% erythrulose | 0 | 310000 | |
| | 24 | 3000 | 2 |
| 0.1 wt.-% retinyl acetate | 0 | 310000 | |
| | 24 | 3000 | 2 |
| 0.05 wt.-% erythrulose | 0 | 310000 | |
| 0.05 wt.-% retinyl acetate | 24 | 550 | 3 |

As can be seen in the table above the combination of erythrulose and retinyl acetate shows a synergistic effect against Propionibacterium acnes.
(The term "Log reduction" is a well-known mathematical term used to show the relative number of cells, germs, microbes, etc., reduced in or on something, e.g. a 2 log reduction means the number of microbes is 100 times smaller and a 3 log reduction means the number of microbes is 1000 times smaller).

**Table 2: Results on E. coli**

| Test solution | Time [h] | Escherichia coli colony count [cfu/ml] | Δ* |
|---|---|---|---|
| 0.1 wt.-% erythrulose | 0 | 250000 | |
| | 24 | 530000 | +112% |
| 0.1 wt.-% retinyl acetate | 0 | 250000 | |
| | 24 | 530000 | +112% |
| 0.05 wt.-% erythrulose | 0 | 250000 | |
| 0.05 wt.-% retinyl acetate | 24 | 300000 | +20% |
| **C1** | 0 | 250000 | |
| | 24 | 530000 | +112% |

| | | | |
|---|---|---|---|
| *Δ = ({microorganism count t = 24h}-{microorganism count t=0} / {microorganism count t=0})*100 | | | |

As can be seen in the table above the combination of erythrulose and retinyl acetate shows a synergistic effect against Escherichia coli by significantly reducing the growth thereof.

## Claims

1. A topical composition comprising erythrulose and retinyl acetate.

2. The topical composition according to claim 1, wherein the amount of erythrulose is selected in the range of 0.01 to 3 wt.-%, preferably in the range of 0.025 to 2 wt.-%, more preferably in the range of 0.04 to 1 wt.-%, most preferably in the range of 0.04 to 0.75 wt.-%, based on the total weight of the composition.

3. The topical composition according to claim 1 or 2, wherein the amount of the retinyl acetate is selected in the range of 0.00001 to 1 wt.-%, preferably in the range of 0.0001 to 0.75 wt.-%, most preferably in the range of 0.001 to 0.5 wt.-%, based on the total weight of the composition.

4. The topical composition according to any one of claims 1 to 3, wherein the composition is a cosmetic or pharmaceutical composition.

5. The topical composition according to claim 4, wherein the composition is a shampoo preparation, a hair conditioner, an O/W emulsion, a W/O emulsion, or a gel.

6. The topical composition according to any one of claim 1 or 5, wherein the composition furthermore comprises water and at least one agent selected from the group consisting of surfactants, emulsifiers, thickeners, and oils.

7. The topical composition according to any one of claims 1 to 6 for use in the treatment, prevention and/or prophylaxis of fungal and/ or bacterial cell induced diseases and/ or disorders.

8. The topical composition according to any one of claims 1 to 6 for use in the treatment, prevention and/or prophylaxis of acne.

9. The topical composition according to anyone of claim 1 to 6 for the use in killing and/ or inhibiting growth of microbial cells, preferably fungal and/ or bacterial cells.

10. The topical composition according to claim 9, wherein the microbial cells are *P*. *acnes, S. epidermis, C. xerosis, A. brasiliensis, C. albicans, P. aeruginosa, E. coli. M. furfur* and/ or *S*. *aureus,* preferably *E. coli* and/ or *P. acnes.*

11. Use of a combination of erythrulose and retinyl acetate for improving preservation of a product selected from the group of cosmetic compositions, household products, plastics, paper and/ or paints

12. A method of preventing microbial decay and breakdown caused by *E*. *coli* of cosmetic compositions, household products, plastics, paper and/ or paints, wherein said method comprises adding to the cosmetic compositions, household products, plastics, paper and/ or paints erythrulose and retinyl acetate.

## Patentansprüche

1. Topische Zusammensetzung, die Erythrulose und Retinylacetat umfasst.

2. Topische Zusammensetzung nach Anspruch 1, wobei die Menge an Erythrulose im Bereich von 0,01 bis 3 Gew.-%, vorzugsweise im Bereich von 0,025 bis 2 Gew.-%, weiter bevorzugt im Bereich von 0,04 bis 1 Gew.-%, ganz besonders bevorzugt im Bereich von 0,04 bis 0,75 Gew.-%, bezogen auf das Gesamtgewicht der Zusammensetzung, ausgewählt ist.

3. Topische Zusammensetzung nach Anspruch 1 oder 2, wobei die Menge des Retinylacetats im Bereich von 0,00001 Gew.-% bis 1 Gew.-%, vorzugsweise im Bereich von 0,0001 Gew.-% bis 0,75 Gew.-%, ganz besonders bevorzugt im Bereich von 0,001 Gew.-% bis 0,5 Gew.-%, bezogen auf das Gesamtgewicht der Zusammensetzung, ausgewählt ist.

4. Topische Zusammensetzung nach einem der Ansprüche 1 bis 3, wobei es sich bei der Zusammensetzung um eine kosmetische oder pharmazeutische Zusammensetzung handelt.

5. Topische Zusammensetzung nach Anspruch 4, wobei es sich bei der Zusammensetzung um eine Shampoozubereitung, ein Haarkonditionierungsmittel, eine O/W-Emulsion, eine W/O-Emulsion oder ein Gel handelt.

6. Topische Zusammensetzung nach 1 oder 5, wobei die Zusammensetzung ferner Wasser und mindestens ein Mittel aus der Gruppe bestehend aus Tensiden, Emulgatoren, Verdickungsmitteln und Ölen umfasst.

7. Topische Zusammensetzung nach einem der Ansprüche 1 bis 6 zur Verwendung bei der Behandlung, Prävention und/oder Prophylaxe von durch Pilz- und/oder Bakteriellenzellen induzierten Erkrankungen und/oder Störungen.

8. Topische Zusammensetzung nach einem der Ansprüche 1 bis 6 zur Verwendung bei der Behandlung, Prävention und/oder Prophylaxe von Akne.

9. Topische Zusammensetzung nach einem der Ansprüche 1 bis 6 zur Verwendung beim Abtöten und/oder Inhibieren des Wachstums von Mikrobenzellen, vorzugsweise Pilz- und/oder Bakterienzellen.

10. Topische Zusammensetzung nach Anspruch 9, wobei es sich bei den Mikrobenzellen um *P. acnes, S. epidermis, C. xerosis, A. brasiliensis, C. albicans, P. aeruginosa, E. coli, M. furfur* und/oder *S. aureus,* vorzugsweise E. *coli* und/oder *P. acnes,* handelt.

11. Verwendung einer Kombination von Erythrulose und Retinylacetat zur Verbesserung der Konservierung eines Produkts aus der Gruppe der kosmetischen Zusammensetzungen, Haushaltsprodukte, Kunststoffe, Papier und/oder Anstrichmittel.

12. Verfahren zur Verhinderung von durch *E*. *coli* verursachtem mikrobiellem Verfalls und Abbau von kosmetischen Zusammensetzungen, Haushaltsprodukten, Kunststoffen, Papier und/oder Anstrichmitteln, wobei das Verfahren das Zugeben von Erythrulose und Retinylacetat zu den kosmetischen Zusammensetzungen, Haushaltsprodukten, Kunststoffen, Papier und/oder Anstrichmitteln umfasst.

## Revendications

1. Composition topique comprenant de l'érythrulose et de l'acétate de rétinyle.

2. Composition topique selon la revendication 1, dans laquelle la quantité d'érythrulose est choisie dans la plage de 0,01 à 3 % en poids, de préférence dans la plage de 0,025 à 2 % en poids, plus préférablement dans la plage de 0,04 à 1 % en poids, le plus préférablement dans la plage de 0,04 à 0,75 % en poids, sur la base du poids total de la composition.

3. Composition topique selon la revendication 1 ou 2, dans laquelle la quantité de l'acétate de rétinyle est choisie dans la plage de 0,00001 à 1 % en poids, préférablement dans la plage de 0,0001 à 0,75 % en poids, le plus préférablement dans la plage de 0,001 à 0,5 % en poids, sur la base du poids total de la composition.

4. Composition topique selon l'une quelconque des revendications 1 à 3, dans laquelle la composition est une composition cosmétique ou pharmaceutique.

5. Composition topique selon la revendication 4, dans laquelle la composition est une préparation de shampooing, un après-shampooing, une émulsion H/E, une émulsion E/H ou un gel.

6. Composition topique selon l'une quelconque des revendications 1 ou 5, dans laquelle la composition comprend en outre de l'eau et au moins un agent choisi dans le groupe constitué par les tensioactifs, les émulsifiants, les épaississants et les huiles.

7. Composition topique selon l'une quelconque des revendications 1 à 6, destinée à être utilisée dans le traitement, la prévention et/ou la prophylaxie de maladies et/ou troubles induits par des cellules fongiques et/ou bactériennes.

8. Composition topique selon l'une quelconque des revendications 1 à 6, destinée à être utilisée dans le traitement, la prévention et/ou la prophylaxie de l'acné.

9. Composition topique selon l'une quelconque des revendications 1 à 6, destinée à être utilisée pour tuer et/ou inhiber la croissance de cellules microbiennes, de préférence de cellules fongiques et/ou bactériennes.

10. Composition topique selon la revendication 9, dans laquelle les cellules microbiennes sont *P. acnes, S. epidermis, C. xerosis, A. brasiliensis, C. albicans, P. aeruginosa, E. coli. M. furfur* and/or *S. aureus,* de préférence *E. coli* et/ou *P. acnes.*

11. Utilisation d'une combinaison d'érythrulose et d'acétate de rétinyle pour améliorer la conservation d'un produit choisi dans le groupe des compositions cosmétiques, des produits ménagers, des plastiques, du papier et/ou des peintures.

12. Procédé de prévention de la désintégration et de la dégradation microbienne provoquée par *E. coli* de compositions cosmétiques, de produits ménagers, de plastiques, de papier et/ou de peintures, dans lequel ledit procédé comprend l'ajout aux compositions cosmétiques, produits ménagers, plastiques, papier et/ou peintures d'érythrulose et d'acétate de rétinyle.
